# EUROPEAN PATENT APPLICATION

(11) **EP 4 834 281 A1**
(43) Date of publication of application: **04.11.2026**
(21) Application number: 24913786.0
(22) Date of filing: 18.11.2024
(51) Int. Cl.: A61F 2/12, A61F 2/00

(54) **ARTIFICIAL BREAST PROSTHESIS DELIVERY DEVICE**

(30) Priority: 29.12.2023 KR 20230196544
(71) Applicant: Osstemimplant Co., Ltd., Gangseo-gu Seoul 07789 (KR)
(72) Inventor: PARK, Ju Yeon, Seoul 07739 (KR); KIM, Jung Ju, Bucheon-si, Gyeonggi-do 14685 (KR); JANG, Il Seok, Yangsan-si, Gyeongsangnam-do 50653 (KR); SONG, Ju Dong, Busan 48272 (KR); EOM, Tae Gwan, Busan 48104 (KR); CHOI, Kyoo Ok, Seoul 07789 (KR)
(74) Representative: Germain Maureau
(86) International application number: PCT/KR2024/096608
(87) International publication number: WO 2025/143982

(57) **Abstract**

A breast implant delivery device (1) includes a tapered body (10) configured to receive a breast implant and having a width that narrows from one side toward the other side, and a plurality of longitudinal grooves (20) recessed in an inner surface of the body (10), extending from the one side to the other side, and arranged to be circumferentially spaced apart from one another.

## Description

### Technical Field

The present invention relates to a breast implant delivery device.

### Background Art

During breast augmentation surgery, skin tissue is incised and an implant is inserted into the body. If a large incision is made in the skin tissue during surgery, the skin tissue at the incision site may be damaged, and scarring and sequelae may occur.

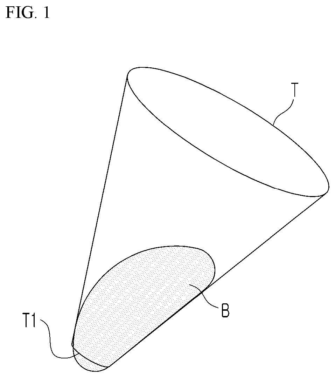

Accordingly, the tapered breast implant delivery device (T) of FIG. 1, which can minimize the incision site during implant insertion, has become widely used. Since the implant (B) can be inserted into the body through a narrowing structure, insertion through a minimal incision is possible, the risk of infection can be reduced, and operation time and recovery time can be shortened.

However, due to the structure of the tapered breast implant delivery device (T), a large insertion force is required for the implant (B) to pass through the narrow opening (T1), and there is concern that side effects may occur if such an insertion force is applied to the implant.

Accordingly, Keller Medical, Inc. has developed the Keller funnel (US 8,211,173 B2), which facilitates sliding from a large end to a small end by smoothing the inner surface of the tapered structure through chemical treatment.

However, since the Keller funnel also has a tapered structure, there remains a limitation in that a large insertion force is required when inserting an implant.

### Documents Cited

### Patent Document

US 8,211,173 B2 (issued July 3, 2012)

### Summary of the Invention

### Technical Problem

The present invention is intended to solve the above-described problems of the prior art, and an object of the present invention is to provide a tapered breast implant delivery device in which the insertion force required for inserting an implant can be reduced.

### Solution

One aspect of the present invention provides a breast implant delivery device including a tapered body configured to receive a breast implant and having a width that narrows from one side toward the other side, and a plurality of longitudinal grooves recessed in an inner surface of the body, extending from one side to the other side, and arranged to be circumferentially spaced apart from one another.

In one embodiment, a groove spacing between each longitudinal groove and an adjacent longitudinal groove may narrow from one side toward the other side.

In one embodiment, the groove spacing at one end of each longitudinal groove may be 0.15 mm or more and 100 mm or less, and the groove spacing at the other end of each longitudinal groove may be 0.025 mm or more and 50 mm or less.

In one embodiment, a depth of each longitudinal groove may be 0.01 mm or more and 0.15 mm or less.

In one embodiment, a length of each longitudinal groove may be 100 mm or more and 400 mm or less.

In one embodiment, the longitudinal grooves may be disposed at equal intervals from adjacent longitudinal grooves.

In one embodiment, the body may be formed of an elastic material.

In one embodiment, hydrophilic functional groups may be formed on an inner surface of the body.

In one embodiment, a lubricant may be coated on an inner surface of the body.

In one embodiment, the body may have a hollow structure and may be formed such that a first opening at one end is larger than a second opening at the other end.

### Advantageous Effects

According to one aspect of the present invention, friction is reduced by the longitudinal grooves formed on the inner surface of the breast implant delivery device, and stress is concentrated on the narrower opening side, so that the insertion force required for inserting an implant can be reduced.

The effects of the present invention are not limited to the above-described effects, and should be understood to include all effects that can be inferred from the detailed description of the present invention or the configuration of the invention described in the claims.

### Brief Description of the Drawings

FIG. 1 illustrates a conventional tapered breast implant delivery device.
FIG. 2 is a view schematically illustrating a breast implant delivery device according to one embodiment of the present invention.
FIG. 3 is a view schematically illustrating a longitudinal cross-sectional view of the breast implant delivery device with the other-end side removed.
FIG. 4 is a view schematically illustrating a part of a transverse cross-sectional view of the breast implant delivery device of FIG. 2.
FIGS. 5 to 7 are graphs showing results of measuring insertion force of breast implant delivery devices according to Examples and Comparative Examples described herein.

### Detailed Description of Embodiments

Hereinafter, the present invention will be described with reference to the accompanying drawings. However, the present invention may be implemented in various different forms and is not limited to the embodiments described herein. In addition, in order to clearly describe the present invention in the drawings, parts irrelevant to the description are omitted, and similar reference numerals are assigned to similar parts throughout the specification.

Throughout the specification, when a certain part is described as being "connected" to another part, this includes not only a case of being "directly connected" but also a case of being "indirectly connected" with another member interposed therebetween. In addition, when a certain part is described as "including" a certain component, this means that other components may be further provided, rather than excluding other components, unless specifically stated to the contrary.

Terms including ordinal numbers such as "first" or "second" used in the present specification may be used to describe various components or steps, but the corresponding components or steps should not be limited by the ordinal numbers. Terms including ordinal numbers should be construed only for the purpose of distinguishing one component or step from another component or step.

When a range of numerical values is described in the present specification, unless a specific range is otherwise described, the value has the precision of the provided significant figures according to standard rules in chemistry for significant figures. For example, 10 includes a range of 5.0 to 14.9, and 10.0 includes a range of 9.50 to 10.49. Hereinafter, embodiments of the present invention will be described in detail with reference to the accompanying drawings.

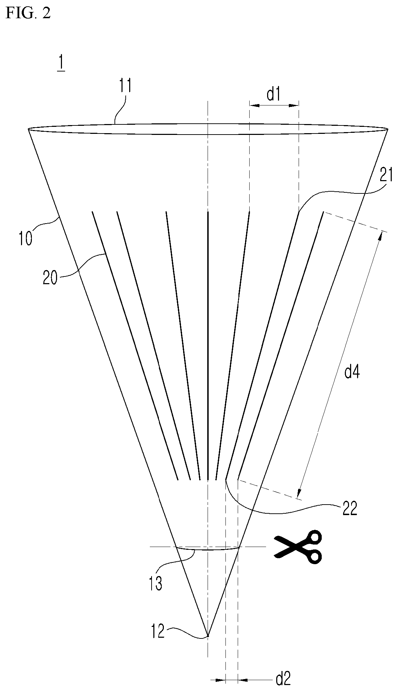

FIG. 2 is a view schematically illustrating a breast implant delivery device according to one embodiment of the present invention.

A breast implant delivery device (1) according to one embodiment of the present invention is configured to deliver a breast implant to a surgical pocket. Here, the surgical pocket refers to an incision site, such as at a patient's axilla or under the breast.

Referring to FIG. 2, the breast implant delivery device (1) according to one embodiment of the present invention includes a body (10) and longitudinal grooves (20).

In detail, the body (10) is formed such that a width of the body (10) narrows toward the other end (12), and a first opening (11) through which an implant is introduced is formed at one end. The pointed other-end (12) side of the body (10) may be cut during implantation surgery of the implant. In this case, a second opening (13) through which the implant can be discharged may be formed on the other side of the body (10), and the second opening (13) may be formed to be smaller than the first opening (11).

For example, the body (10) may have a tapered, conical, or funnel shape. Alternatively, the body (10) may have a pyramidal shape. However, the present invention is not limited thereto, and the body (10) may be provided as a hollow structure in which the opening (11) on one side is larger than the opening (13) on the other side. As such, various shapes in which a width of the body (10) narrows from one side toward the other side may be applied.

When an operator applies a load to the body (10) by means of such a tapered structure of the body (10), the implant may slide along the narrowing inner surface of the body (10) and move to the surgical pocket.

The body (10) may be made of an elastic material. For example, the body (10) may be made of a flexible and transparent material such as polyvinyl chloride (PVC), polyethylene, polyester, or polyurethane. Alternatively, the body (10) may be made of a soft material that can be easily wrinkled, such as vinyl or a thin fabric.

In one embodiment, the inner surface of the body (10) may be provided with a hydrophilic coating.

In one embodiment, the inner surface of the body (10) may be coated with a material having hydrophilic functional groups, such as HA (hyaluronic acid), cellulose, glycerol, ethylene glycol, or sulfonic acid.

In one embodiment, the inner surface of the body (10) may be coated with a hydrophilic lubricant such as cellulose, hyaluronic acid, propylene glycol, glycerol, 4,4'-diaminostilbene-2,2'-disulfonic acid (DASDA), 2'-deoxyadenosine 5'-monophosphate (DAP), 2'-deoxycytidine 5'-monophosphate (DCP), and N-[tris(hydroxymethyl)methyl]-3-aminopropanesulfonic acid.

Accordingly, when the frictional force of the inner surface of the body (10) is reduced and an operator applies pressure to the body (10), the implant may slide along the inner surface of the body (10) and move to the surgical pocket.

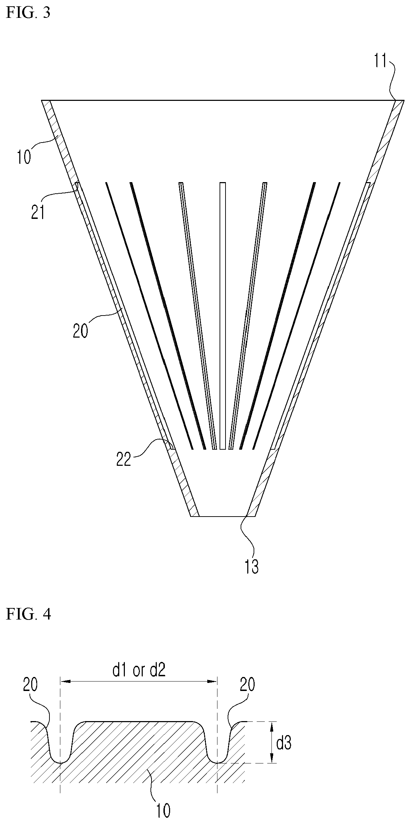

FIG. 3 is a view schematically illustrating a longitudinal cross-sectional view of the breast implant delivery device with the other-end side removed.

Referring to FIG. 3, the longitudinal grooves (20) are recessed to a predetermined depth in the inner surface of the body (10) and extend from one side to the other side of the body (10), that is, in the insertion direction of the implant or in the direction in which a load is applied.

Here, when grooves are formed in a transverse direction perpendicular to the insertion direction of the implant, the load is dispersed along the grooves, and thus the effect of transmitting the load from the first opening (11) toward the second opening (13), that is, stress concentration, is reduced. In addition, the grooves may instead increase the frictional force, thereby increasing the load required for implant insertion.

A plurality of longitudinal grooves (20) may be formed along a circumference of the body (10) and may be disposed to be spaced apart from one another in a circumferential direction. In detail, the plurality of longitudinal grooves (20) may be formed in a state of being circumferentially spaced apart at equal intervals with respect to a center of the body (10). That is, the groove spacing may be constant along the circumference of the body (10).

The groove spacing, which is a circumferential spacing between the longitudinal groove (20) and an adjacent longitudinal groove (20), may be formed to narrow from one side toward the other side.

FIG. 4 is a view schematically illustrating a part of a transverse cross-sectional view of the breast implant delivery device of FIG. 2.

Referring to FIGS. 2 and 4, the groove spacing (d1) on the first opening (11) side, that is, the groove spacing (d1) at one end (21) of the longitudinal groove (20), may be formed to be 0.15 mm or more and 100 mm or less, and the groove spacing (d2) on the second opening (13) side, that is, the groove spacing (d2) at the other end (22) of the longitudinal groove (20), may be formed to be 0.025 mm or more and 50 mm or less. Here, the groove spacing (d1) on the first opening (11) side may be formed to be greater than the groove spacing (d2) on the second opening (13) side.

When the groove spacing (d1) at one end (21) of the longitudinal groove (20) is less than 0.15 mm, or the groove spacing (d2) at the other end (22) of the longitudinal groove (20) is less than 0.025 mm, the strength of the body (10) may be weakened, and the body (10) may be torn. In addition, when the groove spacing (d1) at one end (21) of the longitudinal groove (20) exceeds 100 mm, or the groove spacing (d2) at the other end (22) of the longitudinal groove (20) exceeds 50 mm, the reduction in surface area is insignificant, stress concentration is reduced, and the insertion-force-reducing effect is not observed.

In one embodiment, a depth (d3) of the longitudinal groove (20) may be formed to be 0.01 mm or more and 0.15 mm or less.

When the depth (d3) of the longitudinal groove (20) is less than 0.01 mm, if a load is applied, the longitudinal groove (20) may unfold and disappear, and the effect of stress concentration may disappear. When the depth (d3) of the longitudinal groove (20) exceeds 0.15 mm, the implant may enter the spaces between the longitudinal grooves (20), the contact area between the body (10) and the implant is increased, and, as a result, the frictional force is increased, so that stress concentration may be inhibited.

In one embodiment, a length (d4) of the longitudinal groove (20) may be formed to be 100 mm or more and 400 mm or less.

### Examples

Hereinafter, Examples of the present specification will be described in more detail. However, the following experimental results describe only representative experimental results among the above embodiments, and the scope and content of the present specification should not be interpreted as being reduced or limited by the Examples or the like. Each effect of various embodiments of the present specification not explicitly presented below will be specifically described in the corresponding portion.

The breast implant delivery devices of Examples 1 to 6 were all manufactured using the same elastic material, and the lengths of the longitudinal grooves were formed to be 250 mm. However, the groove spacing at one end of the longitudinal grooves, the groove spacing at the other end of the longitudinal grooves, and/or the depth of the longitudinal grooves were varied as shown in Table 1 below.

The same type of hydrophilic lubricant was uniformly applied to the inner surfaces of the breast implant delivery devices of Examples 2 to 6.

The breast implant delivery device of Comparative Example 1 was manufactured in the same manner as Example 1, except that the longitudinal grooves were omitted.

The breast implant delivery device of Comparative Example 2 was manufactured in the same manner as Example 2, except that the longitudinal grooves were omitted.

**Table 1**

| **Category** | **Groove spacing at one end of longitudinal groove (mm)** | **Groove spacing at other end of longitudinal groove (mm)** | **Depth of longitudinal groove (mm)** | **Hydrophilic lubricant applied** |
|---|---|---|---|---|
| Example 1 | 50 | 20 | 0.15 | X |
| Example 2 | 100 | 40 | 0.01 | O |
| Example 3 | 50 | 20 | 0.01 | O |
| Example 4 | 0.15 | 0.025 | 0.01 | O |
| Example 5 | 50 | 20 | 0.08 | O |
| Example 6 | 50 | 20 | 0.15 | O |
| Comparative Example 1 | - | - | - | X |
| Comparative Example 2 | - | - | - | O |

### Experimental Example 1: Measurement of insertion force according to presence or absence of longitudinal grooves

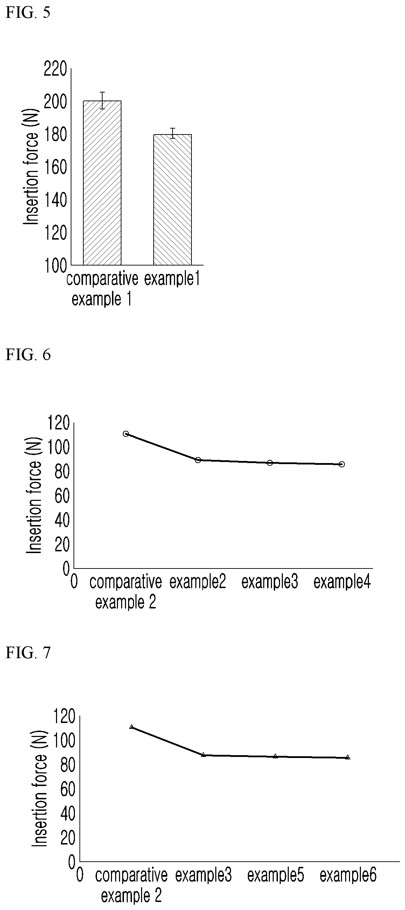

In order to show an effect of reducing insertion force according to the presence or absence of the longitudinal grooves, insertion force was measured in the breast implant delivery devices of Comparative Example 1 and Example 1, and the results are shown in FIG. 5 and Table 2.

In detail, using an Instron universal material testing machine, when the implant was inserted from the first opening toward the second opening in the breast implant delivery device at a constant speed (100 mm/min), the maximum load measured by the universal material testing machine was defined as the insertion force.

**Table 2**

| **Category** | **Insertion force (N)** |
|---|---|
| Comparative Example 1 | 200 |
| Example 1 | 180 |

Referring to FIG. 5 and Table 2, it was confirmed that, in the breast implant delivery device of Example 1 in which the longitudinal grooves are formed, the insertion force required when inserting the implant was reduced by about 10% compared to the breast implant delivery device of Comparative Example 1 having no longitudinal grooves.

### Experimental Example 2: Measurement of insertion force according to spacing of longitudinal grooves

In order to show an effect of reducing insertion force according to the spacing of the longitudinal grooves, insertion force was measured in the breast implant delivery devices of Comparative Example 2 and Examples 2 to 4 in the same manner as in Experimental Example 1, and the results are shown in FIG. 6 and Table 3.

**Table 3**

| **Category** | **Insertion force (N)** |
|---|---|
| Comparative Example 2 | 110 |
| Example 2 | 89 |
| Example 3 | 87 |
| Example 4 | 86 |

Referring to FIG. 6 and Table 3, it was confirmed that, in the breast implant delivery devices of Examples 2 to 4 in which the longitudinal grooves are formed, the insertion force was reduced by about 20% compared to the breast implant delivery device of Comparative Example 2 having no longitudinal grooves. Further, as the spacing of the longitudinal grooves decreased, the insertion force decreased.

For reference, when the groove spacing at one end of the longitudinal grooves was less than 0.15 mm, or the groove spacing at the other end of the longitudinal grooves was less than 0.025 mm, the strength of the body was weakened and the body was torn. In addition, when the groove spacing at one end of the longitudinal grooves exceeded 100 mm, or the groove spacing at the other end of the longitudinal grooves exceeded 50 mm, the reduction in surface area was insignificant, stress concentration was reduced, and the insertion-force-reducing effect was not observed.

### Experimental Example 3: Measurement of insertion force according to depth of longitudinal grooves

In order to show an effect of reducing insertion force according to the depth of the longitudinal grooves, insertion force was measured in the breast implant delivery devices of Comparative Example 2 and Examples 3, 5, and 6 in the same manner as in Experimental Example 1, and the results are shown in FIG. 7 and Table 4.

**Table 4**

| **Category** | **Insertion force (N)** |
|---|---|
| Comparative Example 2 | 110 |
| Example 3 | 87 |
| Example 5 | 86 |
| Example 6 | 85 |

Referring to FIG. 7 and Table 4, it was confirmed that, in the breast implant delivery devices of Examples 3, 5, and 6 in which the longitudinal grooves are formed, the insertion force was reduced by about 20% compared to the breast implant delivery device of Comparative Example 2 having no longitudinal grooves. Further, as the depth of the longitudinal grooves increased, the insertion force decreased.

For reference, when the depth of the longitudinal grooves was less than 0.01 mm, if a load was applied, the longitudinal grooves unfolded and disappeared, stress concentration was reduced, and the insertion-force-reducing effect was not observed. In addition, when the depth of the longitudinal grooves exceeded 0.15 mm, the implant entered the spaces between the longitudinal grooves, the contact area between the body and the implant was increased, and the frictional force was increased thereby, so that stress concentration was reduced and the insertion-force-reducing effect was not observed.

That is, with the breast implant delivery device according to one embodiment of the present invention, friction is reduced by the longitudinal grooves formed on the inner surface of the breast implant delivery device, and stress is concentrated on the narrower opening side, so that the insertion force required when inserting an implant can be reduced.

The above description of the present invention is for illustration, and a person having ordinary skill in the art to which the present invention pertains will understand that the present invention can be easily modified into other specific forms without changing the technical spirit or essential features of the present invention. Therefore, the embodiments described above should be understood as illustrative in all respects and not restrictive. For example, each component described in a single form may be implemented in a distributed manner, and likewise, components described as being distributed may be implemented in a combined form.

The scope of the present invention is indicated by the claims described below, and all changes or modifications derived from the meaning and scope of the claims and equivalents thereof should be construed as being included in the scope of the present invention.

### List of Reference Signs

- 1: Breast implant delivery device
- 10: Body
- 20: Longitudinal groove

## Claims

1. A breast implant delivery device (1) comprising:
a tapered body (10) configured to receive a breast implant and having a width that narrows from one side toward the other side; and
a plurality of longitudinal grooves (20) recessed in an inner surface of the body (10), extending from the one side to the other side, and arranged to be circumferentially spaced apart from one another.

2. The breast implant delivery device according to claim 1, wherein a groove spacing between each of the longitudinal grooves (20) and an adjacent longitudinal groove (20) narrows from the one side toward the other side.

3. The breast implant delivery device according to claim 2, wherein the groove spacing (d1) at one end (21) of each of the longitudinal grooves (20) is 0.15 mm or more and 100 mm or less, and the groove spacing (d2) at the other end (22) of each of the longitudinal grooves (20) is 0.025 mm or more and 50 mm or less.

4. The breast implant delivery device according to claim 1, wherein a depth (d3) of each of the longitudinal grooves (20) is 0.01 mm or more and 0.15 mm or less.

5. The breast implant delivery device according to claim 1, wherein a length (d4) of each of the longitudinal grooves (20) is 100 mm or more and 400 mm or less.

6. The breast implant delivery device according to claim 1, wherein the longitudinal grooves (20) are disposed at equal intervals from adjacent longitudinal grooves (20).

7. The breast implant delivery device according to claim 1, wherein the body (10) is formed of an elastic material.

8. The breast implant delivery device according to claim 1, wherein hydrophilic functional groups are formed on an inner surface of the body (10).

9. The breast implant delivery device according to claim 1, wherein an inner surface of the body (10) is coated with a lubricant.

10. The breast implant delivery device according to claim 1, wherein the body (10) has a hollow structure, and a first opening (11) at one end is formed to be larger than a second opening (13) at the other end.
